# EUROPEAN PATENT APPLICATION

(11) **EP 4 378 468 A1**
(43) Date of publication of application: **05.06.2024**
(21) Application number: 22861531.6
(22) Date of filing: 30.06.2022
(51) Int. Cl.: A61K 35/28, A61P 21/00, A23L 33/10

(54) **COMPOSITION FOR TREATING MUSCLE LOSS-RELATED DISEASES COMPRISING EXOSOMES DERIVED FROM TONSIL MESENCHYMAL STEM CELLS**

(30) Priority: 23.08.2021 KR 20210110937
(71) Applicant: Cellatoz Therapeutics, Inc., Seongnam-si, Gyeonggi-do 13487 (KR)
(72) Inventor: WOO, So-Youn, Seoul 07804 (KR); LEE, Ryung-Ah, Seoul 07985 (KR); RYU, Kyung-Ha, Seoul 07804 (KR); CHO, Kyung-Ah, Seoul 07804 (KR); KWON, Jiyun, Anyang-si Gyeonggi-do 14068 (KR)
(74) Representative: V.O.
(86) International application number: PCT/KR2022/009455
(87) International publication number: WO 2023/027317

(57) **Abstract**

The present disclosure relates to a pharmaceutical composition for preventing, alleviating or treating a muscle loss-related disease, which contains exosomes derived from tonsil mesenchymal stem cells. Since the composition of the present disclosure, which contains a high content of miR-145-5p, restores musculoskeletal loss, has the effect of recovering muscle fiber loss and inhibition of myotube formation, regulates the expression of activin A, which is a negative regulator for muscle mass, enhances the expression of muscle fiber formation-related genes and enhances the expression of muscle atrophy-related genes, it has superior effect of preventing, alleviating and treating a muscle loss-related disease and improving athletic ability.

## Description

### [Technical Field]

The present disclosure relates to a pharmaceutical composition for preventing or treating a muscle loss-related disease, which contains exosomes derived from tonsil mesenchymal stem cells.

The present disclosure also relates to a food composition for preventing or alleviating a muscle loss-related disease, which contains exosomes derived from tonsil mesenchymal stem cells.

### [Background Art]

Skeletal muscle is the largest organ in the human body, accounting for 40-50% of total body weight, and plays an important role in various metabolic functions in the body, including energy homeostasis, heat production, etc. Human muscle decreases by 1 % or more per year after the age of 40. By the age of 80, up to 50% of muscle mass is decreased. The muscle loss in the old age is recognized as the most factor in the reduction of overall body functions. As aging processes, the change in body constitution including muscle and fat contents, skeletal distortion, etc. are recognized. The prevalence of obesity in old age due to muscle loss continues to increase worldwide at over 30%. Abnormality of insulin secretion can cause problems with muscle development because energy cannot be property supplied to cells. Therefore, sarcopenia is increased in diabetic patients than in normal people. In addition, muscle loss increases the risk of arthritis, back pain and chronic pain and may worsen the symptoms of urinary incontinence due to abdominal obesity and injuries due to fracture can lead to death by increasing depression in old age. Therefore, sarcopenia in old age is associated with various diseases and is the major cause of worsening the quality of life.

Muscle loss-related diseases are caused by weakening of skeletal muscle. With gradually impaired walking and mobility functions and difficulty in activities of daily living (ADL), independent living becomes impossible. In addition, since they cause cardiopulmonary dysfunction and other complications, it is important to accurately understand their characteristics.

Sarcopenia refers to a condition in which the amount and function of skeletal muscle decline. Sarcopenia is caused by a variety of causes including aging, hormonal abnormalities, malnutrition, lack of physical activities, inflammation, degenerative diseases, etc. Among them, cancer, aging and lack of sex hormones are known as main causes. As average life expectancy increases worldwide due to the advances in medical technology and the development of various therapeutic agents, the aging population is increasing and, accordingly, it is expected that the demand for the treatment of sarcopenia will continue to increase. In patients with sarcopenia, the number of myoblasts decreases due to the disorder of recruitment, activity or proliferation of satellite cells, which are the stem cells of myoblasts. As the proliferation and differentiation of myoblasts are reduced, the muscular function of patients with sarcopenia declines because of decreased size and number of muscle fibers at a histological level. In the past decade, researches on the epidemiology of sarcopenia have been conducted actively in the United States and Europe. Recently, interest in the clinical importance of sarcopenia is accelerating. The early researches mainly showed that sarcopenia causes decreased quality of life due to general weakness, impaired activity and decreased muscle strength. But, recently published researches have shown that the risk of osteoporotic fracture can increase significantly in addition to the decline in quality of life. In addition, because sarcopenia induces chronic diseases such as diabetes, metabolic syndrome, obesity, chronic renal failure, chronic hepatic failure, etc. and ultimately increases mortality, sarcopenia is attracting attention as a disease that must be treated appropriately. Recently, it has been reported that the likelihood of development of physical disability in sarcopenia patients is increased by about 1.5 to 3.5 times in the United States, causing an annual social cost of 18.5 billion USD. In Korea, according to the National Health and Nutrition Examination Survey, sarcopenia is a very common disease with the prevalence of 42.0% in men and 42.7% in women over the age of 60. In particular, because Korea has the highest aging rate in the world, it is certain to become an important social problem in the future.

Muscle atrophy is a disease in which the mass of muscles of limbs declines. It is known that muscle atrophy is caused by the increased expression of MuRF-1 and Atrogin-1, which leads to the loss of muscle mass by degrading the myosin heavy chains of muscles.

Sarcopenia, which is caused by degeneration of spinal nerves, motor nerves or skeletal muscle fibers associated with muscle loss-related diseases, is one of the representative intractable diseases whose cause has not been identified yet. According to researches so far, it is known that skeletal muscle does not contract due to the degeneration of the motor nerve that induces skeletal muscle contraction or the decreased expression or deformation of proteins involved in muscle contraction. In the long term, the motor nerve or skeletal muscle is transformed into fibrous tissues. Since the fundamental cause of sarcopenia has not been identified yet and the method for preventing or restoring the degeneration of the motor nerve or skeletal muscle has not been developed, researches are being conducted actively to develop a method for slowing the progression of sarcopenia.

At present, it is known that exercise and intake of protein and calories are helpful for sarcopenia. However, they are not so helpful in elderly people, who make up the majority of sarcopenia patients. Therefore, a therapeutic agent for sarcopenia is needed desperately. However, the therapeutic agents currently used for sarcopenia, which exhibit a direct effect on alleviating muscle loss and enhancing muscle mass, are still at the clinical trial level and there is no drug that has been finally approved by the FDA. Therefore, there have been efforts to develop selective androgen receptor modulators, activin receptor antagonists, fast skeletal muscle troponin inhibitors, etc. into therapeutic agents for sarcopenia, but they are currently at the level of early clinical trials. At present, as a method for treating sarcopenia, suppression of muscle atrophy caused by the degeneration or progressive transformation of muscle cells is used mainly.

Tonsil mesenchymal stem cells refer to undifferentiated stem cells having the ability to self-replicate and the ability to differentiate into two or more types of new cells, which are derived from the tonsil, a tissue located at the back of the throat and nose that primarily defends the body from substances invading from outside such as bacteria, etc. and functions as a lymphoid epithelial tissue. Although the therapeutic effect of the tonsil mesenchymal stem cells for inflammatory skin diseases or chronic inflammatory intestinal diseases has been reported, no research has been conducted on exosomes derived therefrom.

Under this background, the inventors of the present disclosure have identified that the exosomes derived from tonsil mesenchymal stem cells have characteristics different from those of the exosomes derived from the mesenchymal stem cells derived from other sources and have completed the present disclosure by identifying that they exhibit superior therapeutic effect for muscle loss-related diseases.

### [Disclosure]

### [Technical Problem]

The present disclosure relates to a pharmaceutical composition for preventing or treating a muscle loss-related disease, which contains exosomes derived from tonsil mesenchymal stem cells.

The present disclosure also relates to a food composition for preventing or alleviating a muscle loss-related disease, which contains exosomes derived from tonsil mesenchymal stem cells.

The present disclosure also relates to a food composition for increasing muscle mass or improving muscular function, which contains exosomes derived from tonsil mesenchymal stem cells.

### [Technical Solution]

The present disclosure provides a pharmaceutical composition for preventing or treating a muscle loss-related disease, which contains exosomes derived from tonsil mesenchymal stem cells.

In the present disclosure, tonsil mesenchymal stem cells refer to undifferentiated stem cells having the ability to self-replicate and the ability to differentiate into two or more types of new cells, which are derived from the tonsil, a tissue located at the back of the throat and nose that primarily defends the body from substances invading from outside such as bacteria, etc. and functions as a lymphoid epithelial tissue.

The tonsil from which the tonsil mesenchymal stem cells according to the present disclosure are derived can be obtained easily as compared to other sources of stem cells. Since the tonsil tissue discarded after surgery can be recycled and the initial yield is very high, the amount of a conditioned medium for extracting the exosomes to be injected can be adjusted freely.

In the present disclosure, the conditioned medium of the tonsil mesenchymal stem cells includes those prepared through isolation from an individual, culturing and special manipulation. The exosomes that can be used in drugs or foods used for the purpose of treatment, diagnosis and prevention can be extracted using the conditioned medium.

In the present disclosure, tonsil mesenchymal stem cells can be extracted from the tonsil according to methods known in the art. For example, they can be extracted from the tonsil according to the methods described in Korean Patent Registration No. 10-1508413, etc.

The conditioned medium of tonsil mesenchymal stem cells may be a culture medium obtained by culturing tonsil mesenchymal stem cells and removing the cells, or a culture supernatant, concentrate or freeze-dried product thereof.

The tonsil mesenchymal stem cells may be cultured using a medium for culturing stem cells. The culture medium of tonsil mesenchymal stem cells may be obtained by culturing tonsil mesenchymal stem cells derived from the tonsil tissue in a serum-containing or serum-free medium. The conditioned medium for extracting exosomes may be centrifuged or filtered to remove stem cells and macromolecules, and the resulting supernatant may be used. The obtained supernatant may be used as it is or after concentration for extraction of the exosomes.

The medium for culturing tonsil mesenchymal stem cells and culturing condition are well known in the art to which the present disclosure belongs and can be selected or modified adequately by those having ordinary knowledge.

In the present disclosure, "exosomes" refer to small vesicles with a membrane structure secreted from various cells. In the research using an electron microscope, it was discovered that the exosomes are not separated directly from the plasma membrane but originate from specific intracellular compartments called multivesicular bodies (MVBs) and are released and secreted out of cells. That is to say, when the multivesicular bodies are fused with the plasma membrane, vesicles called exosomes are secreted to the extracellular environment. Although the molecular mechanism by which these exosomes are produced has not been elucidated clearly, it is known that not only red blood cells but also various types of immune cells such as B lymphocytes, T lymphocytes, dendritic cells, platelets, macrophages, etc., tumor cysts, stem cells, etc. produce and secrete exosomes. The exosomes include those secreted naturally or artificially.

The exosomes according to the present disclosure are exosomes derived from tonsil mesenchymal stem cells.

In the present disclosure, the exosomes derived from tonsil mesenchymal stem cells may have a diameter of 10-300 nm. The exosomes may have a diameter of specifically 40-200 nm, more specifically 50-150 nm.

The exosomes may be positive for CD63 and CD81.

The exosomes may be obtained by an exosome extraction method known in the art. For example, they may be obtained by an extraction method including the following steps:
1) a step of culturing tonsil mesenchymal stem cells;
2) a step of recovering a culture supernatant of the tonsil mesenchymal stem cells;
3) a step of removing cell debris by centrifuging the recovered culture supernatant; and
4) a step of obtaining isolated and purified exosomes from the culture supernatant with the cell debris removed using one of TFF (tangential flow filtration), ultracentrifugation, size-exclusion chromatography and exosome isolation kit.

The tonsil mesenchymal stem cells may be cultured at normal oxygen concentration, i.e., under supply of 21% oxygen (O₂), or under a condition in which a hypoxic cell sensitizer is not added to the cell culture medium, although not being limited thereto.

In the step 1), any cell culture medium commonly used in the art. For example, DMEM (Dulbecco's modified Eagle's medium), MEM (minimal essential medium) or RPMI 1640 (Roswell Park Memorial Institute 1640) may be used, although not being limited thereto.

In addition, one or more auxiliary ingredient may be added to the cell culture medium as needed. The auxiliary ingredient may be one or more selected from a group consisting of fetal bovine, horse or human serum, an antibiotic for preventing microbial contamination, such as penicillin G, streptomycin sulfate, gentamycin, etc., an antifungal agent such as amphotericin B, nystatin, etc. and mixtures thereof.

During the culturing for extracting exosomes from the tonsil mesenchymal stem cells, if necessary, the culture may further comprise replacing with a serum-free, antibiotic-free or phenol red-free medium, and then culturing with the medium. That is to say, the step 2) may be performed after culturing the cells in a normal culture medium and then culturing further after replacing the medium with a serum-free medium, etc.

In the step 2), the culture medium, i.e., the conditioned medium may be recovered. The recovered culture supernatant contains cell debris and exosomes secreted from the stem cells. In the step 3), the cell debris contained in the culture supernatant may be removed by centrifugation.

After the cell debris are removed, isolated and purified exosomes may be obtained using one or more selected from a group consisting of TFF (tangential flow filtration), ultracentrifugation, size-exclusion chromatography and exosome isolation kit.

The exosomes according to the present disclosure contain a large quantity of miR-145-5p unlike exosomes derived from other mesenchymal stem cells.

hsa-miR-145-5p has a base sequence of SEQ ID NO 1:
GUCCAGUUUUCCCAGGAAUCCCU

That is to say, the exosomes in the pharmaceutical composition according to the present disclosure may contain hsa-miR-145-5p of SEQ ID NO 1.

In the present disclosure, the term "prevention" refers to any action of suppressing or delaying the onset of a muscle loss-related disease by administering the exosomes derived from tonsil mesenchymal stem cells according to the present disclosure. In the present disclosure, the term "treatment" refers to any action of improving or beneficially changing the symptoms of a muscle loss-related disease by administering the exosomes derived from tonsil mesenchymal stem cells according to the present disclosure. In the present disclosure, the term "alleviation" refers to any action of improving the bad condition of a muscle loss-related disease by administering the composition of the present disclosure to a subject.

'Muscle cells' collectively refer to the cells that make up the muscle of mammals including human. They may refer to myocytes, myotubes, muscle fibers or all of these. 'Muscle cell differentiation' may refer to the production of muscle cells (myocytes, myotubes and/or muscle fibers) from myoblasts or a series of processes comprising the production.

The composition according to the present disclosure exhibits an improved therapeutic and prophylactic effect for symptoms and diseases related with muscle cell loss.

In the present disclosure, the muscle loss-related disease includes all the symptoms related with muscle loss and diseases caused thereby and refers to a muscle loss-related disease caused by decline in muscular function, muscle wasting, muscle degeneration, etc. That is to say, the muscle loss-related disease may indicate symptoms of muscle loss caused by decline in muscular function, muscle wasting, muscle degeneration, etc.

More specifically, the muscle loss-related disease may be one or more selected from a group consisting of sarcopenia, muscle atrophy, myasthenia, muscular dystrophy, myotonia, hypotonia, muscular weakness, amyotrophic lateral sclerosis and cachexia.

In an exemplary embodiment of the present disclosure, the exosomes derived from tonsil mesenchymal stem cells exhibit the characteristics of exosomes by expressing CD63 and CD81.

In an exemplary embodiment of the present disclosure, the exosomes derived from tonsil mesenchymal stem cells have a diameter smaller than 100 nm.

In an exemplary embodiment of the present disclosure, the exosomes derived from tonsil mesenchymal stem cells contain has-miR-145-5p in large quantities as compared to mesenchymal stem cells derived from other sources.

In an exemplary embodiment of the present disclosure, the exosomes derived from tonsil mesenchymal stem cells exhibit the effect of recovering body weight decrease and/or musculoskeletal loss in a muscle loss-related disease animal model.

In an exemplary embodiment of the present disclosure, the exosomes derived from tonsil mesenchymal stem cells may increase the mass, volume and/or density of musculoskeletal cells.

In an exemplary embodiment of the present disclosure, the exosomes derived from tonsil mesenchymal stem cells enhance the differentiation of muscle cells.

In an exemplary embodiment of the present disclosure, the exosomes derived from tonsil mesenchymal stem cells exhibit the effect of restoring the loss of muscle fibers and increased internalization of nuclei, which are histological characteristics seen in a muscle loss-related disease.

In an exemplary embodiment of the present disclosure, the exosomes derived from tonsil mesenchymal stem cells act as a negative regulator for muscle mass and suppress the expression of activin A that may affect the differentiation of myotubes.

In an exemplary embodiment of the present disclosure, the exosomes derived from tonsil mesenchymal stem cells suppress the expression of MuRF1 and Atrogin1 known as muscle atrophy genes.

In an exemplary embodiment of the present disclosure, the exosomes derived from tonsil mesenchymal stem cells contain has-miR-145-5p in large quantities and can regulate the activity of activin A by binding directly to ACVR2A (activin A receptor type 2A) and ACVR1B (activin A receptor type 1B) which are activin A receptors via the has-miR-145-5p.

In an exemplary embodiment of the present disclosure, the exosomes derived from tonsil mesenchymal stem cells may enhance myotube formation in muscle cells.

In an exemplary embodiment of the present disclosure, the exosomes derived from tonsil mesenchymal stem cells may enhance muscle fiber formation by increasing the expression of MyoD and MYH1 which are associated with muscle fiber formation.

In an exemplary embodiment of the present disclosure, the exosomes derived from tonsil mesenchymal stem cells may decrease the activity of activin A by suppressing the expression of ACVR2A and ACVR1B.

The pharmaceutical composition of the present disclosure may be prepared into various formulations such as a liquid, a suspension, an emulsion, a lotion, an ointment, a freeze-dried formulation, etc. according to common methods.

The pharmaceutical composition of the present disclosure may be formulated into a unit dosage form of a pharmaceutical formulation suitable for administration to a patient in accordance with a conventional method in the pharmaceutical field. The formulation may be administered once or several times. Formulations suitable for such purposes include an injection, an infusion, etc. In addition, the pharmaceutical composition may contain common inert carriers and diluents which are pharmaceutically acceptable. The pharmaceutically acceptable carriers and diluents that may be contained in the pharmaceutical composition of the present disclosure include, but are not limited to, excipients such as starch, sugars and mannitol, fillers and extenders such as calcium phosphate, etc., cellulose derivatives such as carboxymethyl cellulose, hydroxypropyl cellulose, etc., binders such as gelatin, alginate, polyvinylpyrrolidone, etc., lubricants such as talc, calcium stearate, hydrogenated castor oil and polyethylene glycol, disintegrants such as povidone and crospovidone and surfactants such as polysorbate, cetyl alcohol, glycerol, etc. The pharmaceutically acceptable carriers and diluents may be those biologically and physiologically compatible with a recipient. The diluents may be saline, an aqueous buffer, a solvent and/or a dispersion medium, although not being limited thereto. In addition, for example, an injection may further contain a preservative, an analgesic, a solubilizer, a stabilizer, etc. and a formulation for topical application may further contain a base, an excipient, a lubricant, a preservative, etc. The composition of the present disclosure may be used without being frozen or may be frozen for future use. If it is to be frozen, a standard cryopreservative (e.g., DMSO, glycerol or Epilife^{®} cell freezing medium (Cascade Biologics)) may be added in advance.

The composition can be administered by a method commonly used in the art. Specifically, it can be administered directly to a disease site of a patient requiring treatment, although not being limited thereto. In addition, the administration can be made by non-surgical administration using a catheter or by surgical administration such as injection after incision of the disease site. The composition may be administered once or several times a day at a dosage of 10 µL to 1 mL/kg body weight although the administration dosage can vary depending on the degree of concentration. For example, the exosomes may be contained in the composition of the present disclosure in an amount of approximately 1-150 µg/mL or 1-100 µg/mL. In addition, the exosomes may be contained in an amount of, for example, 1×10⁷ to 1×10¹² particles/mL, and the administration dosage may be changed appropriately depending on the subject. It should be understood that the actual administration dosage of the active ingredient should be determined in light of various related factors such as the disease to be treated, the severity of the disease, administration route, the body weight, age and sex of a patient, etc. Therefore, the administration dosage described above does not limit the scope of the present disclosure by any means.

The present disclosure also provides a method for treating a muscle loss-related disease, which includes a step of administering exosomes derived from tonsil mesenchymal stem cells to a subject in need thereof.

The present disclosure also provides a method for enhancing or improving muscle exercise ability, which includes a step of administering exosomes derived from tonsil mesenchymal stem cells to a subject in need thereof.

The present disclosure also provides a method for suppressing muscle loss or increasing muscle mass, which includes a step of administering exosomes derived from tonsil mesenchymal stem cells to a subject in need thereof.

The terms such as "exosomes derived from tonsil mesenchymal stem cells", "muscle loss-related disease", "administration", etc. are the same as described above.

The subject refers to an animal, typically a mammal that can show a beneficial effect from treatment using the exosomes derived from tonsil mesenchymal stem cells of the present disclosure. As a specific example, the subject may include a primate such as human. In addition, the subject may include any subject who has or is at the risk of having the symptoms of a muscle loss-related disease.

The present disclosure also provides a use of exosomes derived from tonsil mesenchymal stem cells for preparation of a drug for treating a muscle loss-related disease.

The present disclosure also provides a composition containing exosomes derived from tonsil mesenchymal stem cells for use in treatment of a muscle loss-related disease.

The present disclosure also provides a use of exosomes derived from tonsil mesenchymal stem cells for treatment of a muscle loss-related disease.

The present disclosure also provides a food composition for preventing or alleviating a muscle loss-related disease, which contains exosomes derived from tonsil mesenchymal stem cells.

The present disclosure also provides a food composition for increasing muscle mass or improving muscular function, which contains exosomes derived from tonsil mesenchymal stem cells.

In the present specification, the 'enhancement or improvement of muscle exercise ability' may refer to the recovery and/or improvement of lost or reduced muscle exercise ability, and/or the enhancement of muscle exercise ability.

The "muscle" comprehensively refers to tendons and muscles and the "muscular function" refers to the ability of a muscle to exert force through contraction, including muscle strength which is the ability of a muscle to exert maximal contraction force to overcome resistance, muscular endurance which is the ability of repeating contraction and relaxation for a given weight, and agility which is the ability of exerting strong force within short time. The muscular function is controlled by the liver and is proportional to muscle mass. The term "improvement of muscular function" means that the muscular function improves better than before.

Exercise performance refers to the ability of performing physical activities such as running, jumping, throwing, swimming, etc. in daily lives or sports quickly, strongly, accurately, long and skillfully. The exercise performance may be defined by factors such as muscle strength, agility, endurance, etc. The enhancement of exercise performance refers to the effect of enhancing muscular function such as muscle strength or muscular endurance, specifically increasing muscle strength or muscular endurance. In addition, it may include the effect of increasing exercise endurance, enhancing muscle strength, improving balance and/or enhancing exercise adaptation.

The composition of the present disclosure may be used in common foods.

The food composition of the present disclosure may be used as a health functional food. The term "health functional foods" means foods manufactured (including processing) with functional raw materials or ingredients beneficial to human health according to the Act on Health Functional Food. The term "functionality" means controlling nutrients for the structure or functions of the human body or providing beneficial effects to health purposes, such as physiological effects.

The food composition of the present disclosure may contain common food additives. The suitability as the "food additive" is determined by the general provisions and test methods approved by the Ministry of Food and Drug Safety unless specified otherwise.

The food additives may be, for example, synthetic chemicals such as ketones, glycine, potassium citrate, nicotinic acid, cinnamonic acid, etc., natural additives such as persimmon pigment, licorice extract, crystalline cellulose, kaoliang pigment, guar gum, etc., or mixed additives such as sodium L-glutamate, alkali additives for noodles, preservatives, tar pigments, etc.

The food composition of the present disclosure may contain 0.01-95 wt%, specifically 5-90 wt%, of exosomes derived from tonsil mesenchymal stem cells based on the total weight of the composition.

In addition, the food composition of the present disclosure may be prepared and processed into a tablet, a capsule, a powder, a granule, a liquid, a pill, etc.

For example, a hard capsule may be prepared by filling a mixture of the exosomes of the tonsil mesenchymal stem cells according to the present disclosure and additives such as an excipient, etc. in a common hard capsule, and a soft capsule may be prepared by filling a mixture of the food composition according to the present disclosure and additives such as an excipient, etc. in a gelatin capsule. The soft capsule may contain a plasticizer such as glycerin, sorbitol, etc., a coloring agent, a preservative, etc., if necessary.

The definitions of the terms excipients, binders, disintegrants, lubricants, flavor enhancers, flavoring agents, etc. are described in literature known in the art and include the same or similar functions (Korean Pharmacopoeia Commentary, Moonseongsa, Korean Association of Colleges of Pharmacy, 5th edition, p33-48, 1989).

The foods are not specially limited in type and include all common health functional foods.

### [Advantageous Effects]

The exosomes derived from tonsil mesenchymal stem cells of the present disclosure, which contain miR-145-5p at a high content, exhibits superior effect of preventing, alleviating and treating a muscle loss-related disease and improving athletic ability by restoring musculoskeletal loss, restoring muscle fiber loss and myotube formation, regulating the expression of activin A, a negative regulator for muscle mass, enhancing the expression of muscle fiber formation-related genes, and decreasing the expression of muscle atrophy-related genes.

### [Brief Description of Drawings]

FIG. 1 shows a result of immunoblotting for exosome markers of exosomes derived from tonsil mesenchymal stem cells.
FIGS. 2A and 2B show a result of identifying the size of exosomes derived from tonsil mesenchymal stem cells by scanning electron microscopy (SEM) and transmission electron microscopy (TEM).
FIG. 3 shows a result of identifying the change in body weight depending on administration of exosomes derived from tonsil mesenchymal stem cells (T-MSC exosomes) in a Bu-Cy-treated animal model.
FIG. 4 shows a result of identifying the change in the mass of the quadriceps muscle depending on administration of exosomes derived from tonsil mesenchymal stem cells (T-MSC exosomes) in a Bu-Cy-treated animal model.
FIG. 5 shows a result of identifying histological change depending on administration of exosomes derived from tonsil mesenchymal stem cells (T-MSC exosomes) in a Bu-Cy-treated animal model.
FIGS. 6A to 6C show a result of identifying the change in the expression of activin A, Murf1 and Atrogin 1 depending on administration of exosomes derived from tonsil mesenchymal stem cells (T-MSC exosomes) in a Bu-Cy-treated animal model.
FIG. 7 shows the effect of suppression of myotube formation in C2C12 cells depending on treatment with activin A.
FIG. 8 shows a result of identifying the change in the expression of MyoD, Myh1, MuF1 and Atrogin1 in C2C12 cells depending on treatment with activin A.
FIGS. 9A and 9B show a result of identifying the expression level of miR-145-5p in exosomes derived from tonsil mesenchymal stem cells (T-MSC exosome) and/or exosomes of Wharton's jelly-derived mesenchymal stem cells (WJ-MSC exosomes).
FIGS. 10A and 10B show a result of identifying the applicability of ACVR2A and ACVR1B by predicting the target sequences of miR-145-5p.
FIG. 11 shows a result of identifying the cytotoxicity of exosomes derived from tonsil mesenchymal stem cells (T-MSC exosome) in C2C12 cells.
FIG. 12 shows a result of identifying the change in myotube formation in C2C12 cells depending on treatment with exosomes derived from tonsil mesenchymal stem cells (T-MSC exosome) and activin A.
FIG. 13 shows a result of identifying the change in the expression of Myh1, ACVR2A and ACVR1B in C2C12 cells depending on treatment with exosomes derived from tonsil mesenchymal stem cells (T-MSC exosome) and activin A.
FIG. 14 shows a result of identifying the change in myotube differentiation in C2C12 cells depending on treatment with exosomes derived from tonsil mesenchymal stem cells (T-MSC exosome) and a miR-145-5p inhibitor.
FIG. 15 shows a result of identifying the change in the expression of ACVR2A, ACVR1B, MyoD, Myh1 and Atrogin 1 in C2C12 cells depending on treatment with exosomes derived from tonsil mesenchymal stem cells (T-MSC exosome) and a miR-145-5p inhibitor.
FIG. 16 shows a result of identifying the change in body weight in a Bu-Cy-treated animal model depending on treatment with exosomes derived from tonsil mesenchymal stem cells (T-MSC exosome) and a miR-145-5p inhibitor.
FIG. 17 shows a result of identifying the change in the mass of the quadriceps muscle in a Bu-Cy-treated animal model depending on treatment with exosomes derived from tonsil mesenchymal stem cells (T-MSC exosome) and a miR-145-5p inhibitor.
FIG. 18 shows a result of identifying histological change in a Bu-Cy-treated animal model depending on treatment with exosomes derived from tonsil mesenchymal stem cells (T-MSC exosome) and a miR-145-5p inhibitor.
FIG. 19 shows a result of identifying the change in the expression of Murf1, Atrogin 1, ACVR2A and ACVR1B in a Bu-Cy-treated animal model depending on treatment with exosomes derived from tonsil mesenchymal stem cells (T-MSC exosome) and a miR-145-5p inhibitor.

### [Best Mode]

Hereinafter, the present disclosure is described through examples and test examples. However, the following examples and test examples are provided only for better understanding of the present disclosure and the present disclosure is not limited by the examples and test examples.

### <Example 1> Isolation of exosomes from tonsil mesenchymal stem cells

A tonsil-derived mesenchymal stem cell conditioned medium (T-CM) was prepared for isolation of exosomes. Established tonsil-derived mesenchymal stem cells (T-MSCs) were cultured on a 100-mm tissue culture plate to approximately 80-90% confluency. The cells were washed 4 times with phosphate-buffered saline (PBS) and the medium was replaced with serum-free DMEM. After culturing for 48 hours, the medium was centrifuged at 1,300 rpm for 5 minutes and then filtered through a 0.2-µm filter. The conditioned medium (CM) was concentrated to 20 times of the initial concentration through centrifugal filtration (cut-off of 3K, Amicon Ultra-15, Millipore, Bedford, MA, USA). 1/5 volume of the ExoQuick-TC reagent (System Biosciences, Palo Alto, CA, USA) was added to the T-CM and mixed. After culturing overnight at 4 °C, the mixture was centrifuged at 4 °C and 1,500xg for 30 minutes. After removing the supernatant, followed by final centrifugation at room temperature for 5 minutes, the exosome pellets were resuspended in PBS and stored at -80 °C after quantification using a BCA protein assay kit (Thermo Fisher Scientific, Waltham, MA, USA).

### <Example 2> Identification of exosomes isolated from tonsil mesenchymal stem cells

The exosomes prepared in Example 1 were identified by immunoblotting of exosome markers, scanning electron microscopy (SEM) and transmission electron microscopy (TEM).

Specifically, immunoblotting was performed on the exosomes derived from tonsil mesenchymal stem cells and a tonsil mesenchymal stem cell lysate to identify the expression of the exosome markers CD63 and CD81. After loading 5 µg of exosomes and tonsil mesenchymal stem cell proteins per lane, the blotted membrane was incubated overnight with antibodies against CD63 (ab193349, Abcam, Cambridge, UK) and CD81(sc-166029, Santa Cruz Biotechnology, Santa Cruz, CA, USA). After washing, the membrane was incubated with secondary antibodies (anti-mouse IgG, Sigma Aldrich). The images were developed using the SuperSignal West Femto substrate (Thermo Fisher Scientific) and scanned using ImageQuant LAS 3000 (GE Healthcare, Little Chalfont, UK).

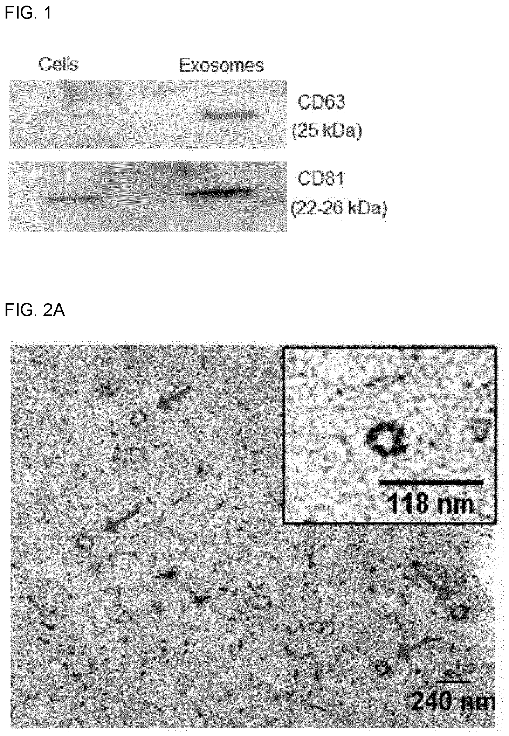

The result is shown in FIG. 1.

As can be seen from FIG. 1, the exosomes derived from tonsil mesenchymal stem cells isolated by the method of the present disclosure expressed CD63 and CD81 and showed the characteristics of exosomes.

In addition, for identification by scanning electron microscopy (SEM), diluted exosomes were dropped on a poly-L-lysine cover glass and prefixed with 0.25% glutaraldehyde for 30 minutes. After washing several times with PBS, the sample was maintained in 1% osmium tetroxide for 30 minutes for final fixation. Then, the sample was serially diluted with ethanol, and washed and dehydrated by critical point drying (CPD). Finally, the sample was mounted onto a stub, sputter-coated with gold and examined with a Sigma-300 microscope (Zeiss, Germany).

In addition, for identification by transmission electron microscopy (TEM), purified exosomes were diluted to 1:1000 in PBS. 5 µL of diluted exosomes were dropped on Formvar-carbon-coated electron microscope (EM) grids. The grids were stained with 2% uranyl acetate and removed using filter paper. Finally, the grids were observed at 80 kV using H-7650 TEM (Hitachi, Tokyo, Japan). Digital images were captured at a magnification of X 70,000-200,000.

The result is shown in FIGS. 2A (SEM) and 2B (TEM).

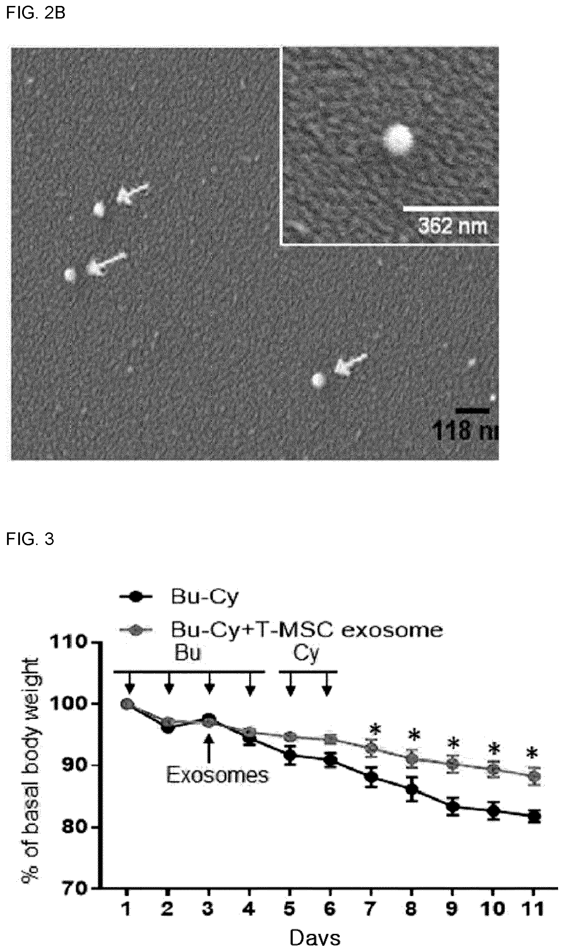

As shown in FIGS. 2A and 2B, the exosomes according to the present disclosure had a diameter smaller than 100 nm as identified by SEM and TEM.

### <Example 2> Identification of therapeutic effect in muscle loss model depending on treatment with T-MSC exosomes

8-week-old female BALB/c mice were purchased from OrientBio (Eumseong-gun, Korea). All animals were housed at 21-23 °C and 51-54% humidity under a pathogen-free environment on a 12/12-hour light/dark cycle and were allowed free access to food and water. The experimental procedures were approved by the Animal Care and Use Committee of the College of Medicine, Ewha Womans University (EUM 20-015).

The female BALB/c mice received busulfan (Bu, 20 mg/kg/day) daily for 4 days, followed by cyclophosphamide (Cy, 100 mg/kg/day) daily for 2 days via intraperitoneal injection. On day 3 during chemotherapy, mice were injected with the exosomes derived from tonsil mesenchymal stem cells (30 µg/mouse) via lateral tail vein. The mice were sacrificed 4-5 days after the last cyclophosphamide injection. All mice were examined daily for the experimental period and weight loss was recorded. At the end of the experimental periods, mice were sacrificed, quadricep masses were measured, and processed for further evaluation including histology and gene expression.

The result is shown in FIGS. 3-6.

FIG. 3 shows the change in the body weight of the mice throughout the experimental period. As can be seen from FIG. 3, rapid weight loss was observed until 4 days post-Bu-Cy injection, but T-MSC exosome injected Bu-Cy mice showed significantly milder weight loss.

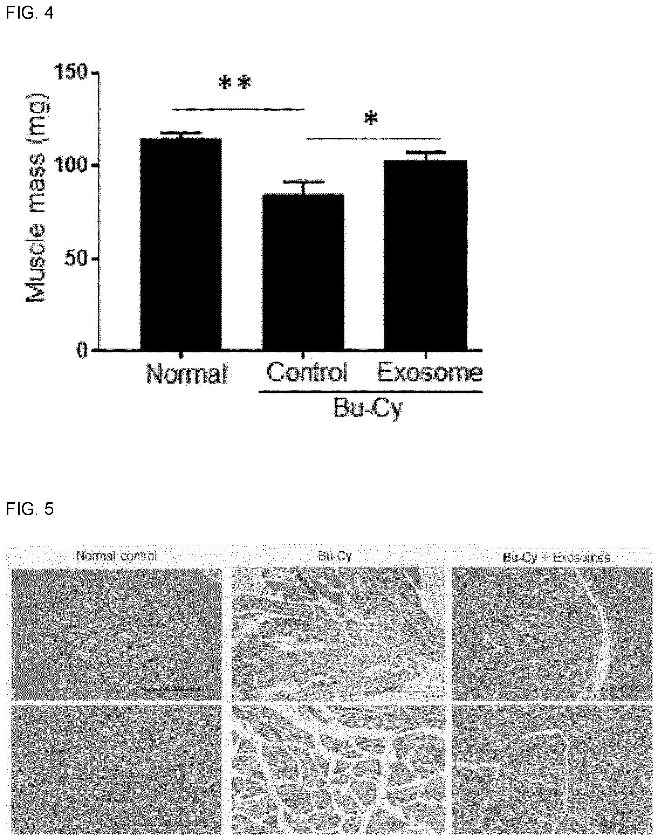

FIG. 4 shows the result of measuring the mass of the quadriceps muscle to investigate skeletal muscle loss. The muscle mass was decreased by the Bu-Cy treatment, but muscle loss induced by chemotherapy was restored significantly by the exosomes according to the present disclosure.

FIG. 5 shows the result of histological analysis. Specifically, Specimens of the quadriceps muscle of the mice were fixed with 4% formaldehyde and embedded in paraffin. The prepared sections (5 µm thickness) were mounted on a slide and stained with hematoxylin and eosin (H&E) for histological evaluation. As can be seen from FIG. 5, the quadriceps muscle tissue in the Bu-Cy mice exhibited the loss of muscle fibers and increased internalization of nuclei, but the exosomes derived from tonsil mesenchymal stem cells restored the muscle loss.

In addition, since activin A is a negative regulator of muscle mass and an increased level of activin A has been reported during chemotherapy, the change in the expression thereof was investigated as well as MuRF1 and Atrogin1, which are known as muscle atrophy genes. Specifically, in order to detect circulating activin A, serums were collected from the normal BALB/c "mice", the Bu-Cy-treated "mice" and the exosome-injected Bu-Cy "mice" at the end of the experiment. The level of activin A was determined using the human/rat/mouse activin A immunoassay kit (R&D Systems, Minneapolis, MN, USA) according to the manufacturer's instructions.

In addition, for detection of muscle-related genes, the quadriceps muscle of the experimental mice was collected, followed by extraction of RNAs using TRIzol (Thermo Fisher Scientific). Then, complementary DNAs were synthesized using a reverse transcription reagent (ELPIS-Biotech, Daejeon, Korea) according to the manufacturer's instructions. Real-time PCR analysis was performed as described previously. All gene expression values (MuRF1 and Atrogin1) were normalized to the GAPDH reference gene.

The sequences of primers used in the experiment are as follows:

| Primer | Sequence | Size (bp) |
|---|---|---|
| *Murf1* | F: 5'-gtgtgaggtgcctacttgctc-3' (SEQ ID NO: 2) | 162 |
| | R: 5'-tgagagatgatcgtctgcact-3' (SEQ ID NO: 3) | |
| *Atrogin1* | F: 5'-cagcttcgtgagcgacctc-3' (SEQ ID NO: 4) | 244 |
| | R: 5'-ggcagtcgagaagtccagtc-3' (SEQ ID NO: 5) | |
| *Gapdh* | F: 5'-ggtaaagtggatattgttgccatcaatg-3' (SEQ ID NO: 6) | 173 |
| | R: 5'-ggtaaagtggatattgttgccatcaatg-3' (SEQ ID NO: 7) | |

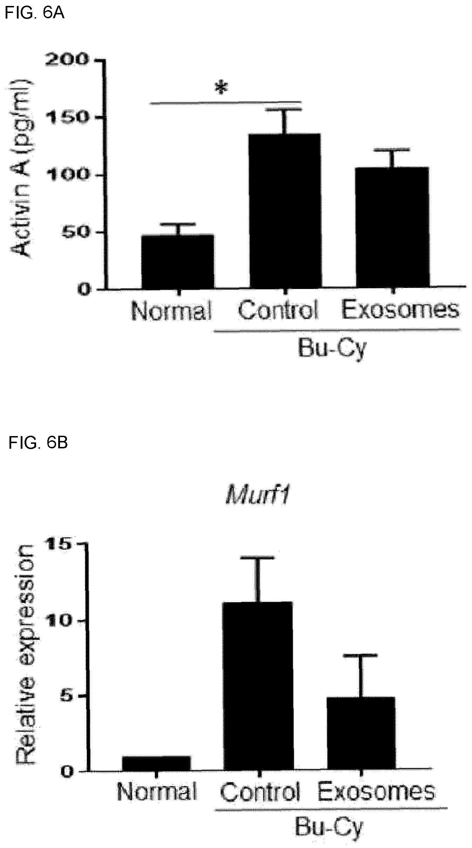

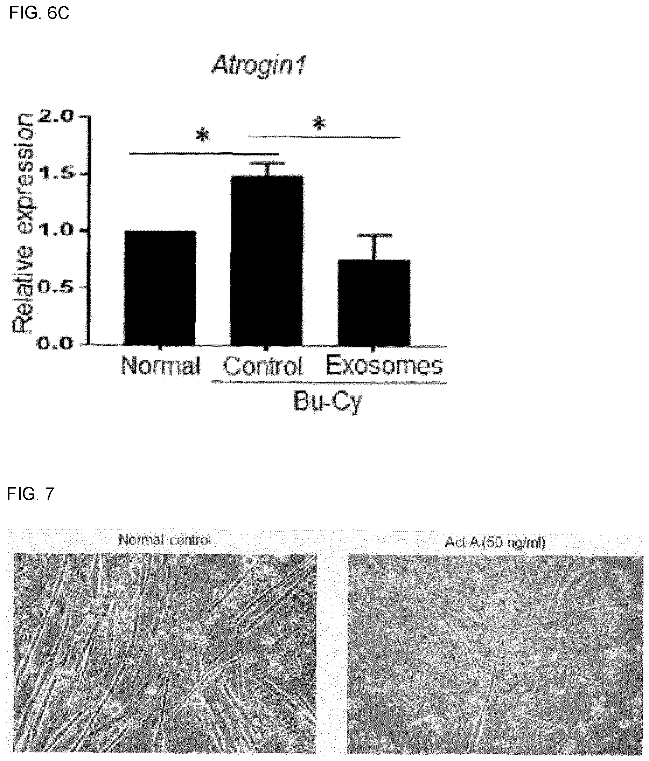

The result is shown in FIG. 6. As can be seen from FIG. 6A, the treatment with Bu-Cy significantly increased circulating activin A. In addition, the expression of MuRF1 and Atrogin1 known as muscle atrophy genes was also increased (FIGS. 6B and 6C). In contrast, the treatment with the exosomes significantly decreased the expression of MuRF1 and Atrogin1.

### <Example 3> Inhibition of myotube differentiation by activin A

It was confirmed above that activin A affects the expression of MuRF1 and Atrogin1. Therefore, the effect of activin A on differentiation ability of C2C12 cells into myotubes was investigated.

Specifically, C2C12 mouse myoblasts were cultured in a growth medium consisting of Dulbecco's modified Eagle's medium (DMEM; Welgene, Daegu, Korea) supplemented with 20% fetal bovine serum (FBS; Welgene), 100 U/mL penicillin and 100 µg/mL streptomycin under the condition of 5% CO₂ and 37 °C. At 90% confluency, the cells were differentiated into myotubes by culturing in DMEM containing 2% horse serum (HS; Sigma-Aldrich, St. Louis, MO, USA), penicillin and streptomycin for 5-7 days. In order to observe the effect of activin A on the differentiation of the C2C12 cells, the cells were treated with 50 ng/mL activin A on the first day of differentiation period.

As can be seen from FIG. 7, when 50 ng/mL activin A was added during the differentiation period, muscle fiber formation was decreased as compared to the untreated C2C12 cells of the control group.

In addition, the change in the expression of MyoD, Myh1, MurF1, Atrogin 1, etc. was investigated by real-time PCR analysis similarly to Example 2.

The sequences of primers further used in addition to those described in Example 2 are as follows:

| Primer | Sequence | Size (bp) |
|---|---|---|
| *MyoD* | F: 5'-ccactccgggacatagacttg-3' (SEQ ID NO: 8) | 109 |
| | R: 5'-aaaagcgcaggtctggtgag-3' (SEQ ID NO: 9) | |
| *Myh1* | F: 5'-gcgaatcgaggctcagaacaa-3' (SEQ ID NO: 10) | 138 |
| | R: 5'-gtagttccgccttcggtcttg-3' (SEQ ID NO: 11) | |

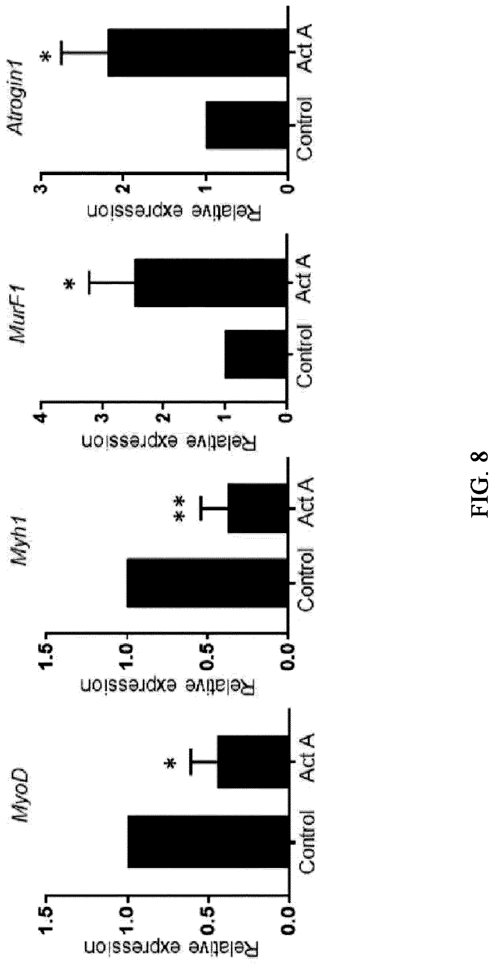

The result is shown in FIG. 8.

As can be seen from FIG. 8, in the cells where fiber formation was not achieved well due to the treatment with activin A, the expression of MyoD and MYH1 was significantly low. In addition, the expression of MuRF1 and Atrogin1 was upregulated by the treatment with activin A.

These results confirm that activin A affects the differentiation of myotubes.

### <Example 4> The expression of has-miR-145-5p in the exosomes derived from tonsil mesenchymal stem cells

Since the exosomes derived from tonsil mesenchymal stem cells effectively recovered the expression of muscle loss-related genes in vivo, it was speculated that the components of exosomes such as miRNA might act on the regulation of muscle mass.

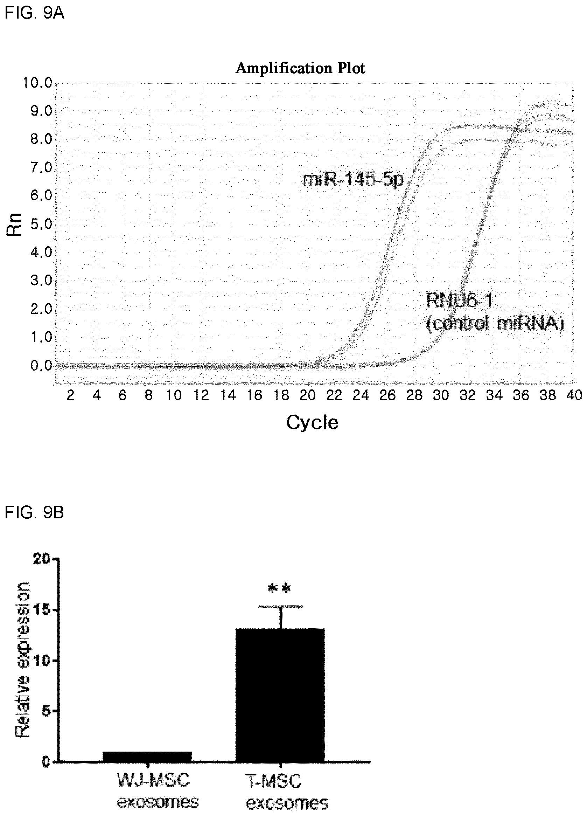

Therefore, microRNAs (miRNAs), exosomal RNAs, were isolated from the T-CMs using the Exo2D for RNA assay kit (Exosome Plus, Gyenggi-do, Korea) according to the manufacturer's instructions. Then, the miRNAs were polyadenylated and then converted to complementary DNAs (cDNAs) using the MystiCq MicroRNA cDNA synthesis mix kit (Sigma-Aldrich) according to the manufacturer's instructions. Real-time PCR analysis was conducted on the StepOnePlus instrument (Applied Biosystems, Foster City, CA, USA) using the SensiFAST SYBR Hi-ROX kit (Bioline, London, UK). The expression of the microRNA-145-5p was normalized to RNU6-1 as a control miRNA. The result is shown in FIG. 9A.

For comparison, the exosomes extracted from Wharton's jelly-derived mesenchymal stem cells in the same manner were used as a comparative example.

The result is shown in FIG. 9B.

As can be seen from FIG. 9B, the exosomes derived from tonsil mesenchymal stem cells including exosomal miRNA, has-miR-145-5p, showed a unique profile.

That is to say, the exosomes derived from tonsil mesenchymal stem cells showed constitutive expression of has-miR-145-5p and the expression was significantly higher as compared to the exosomes of the Wharton's jelly MSCs.

In order to further explore the molecular mechanism that may underlying the regulation of muscle mass, the miR-145-5p target sequence was predicted using the TargetScan Human 7.2 database (http://www.targetscan.org/vert_72/).

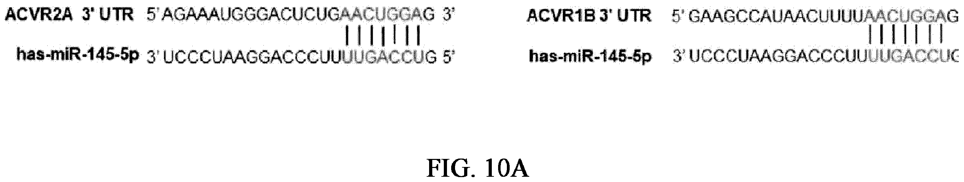

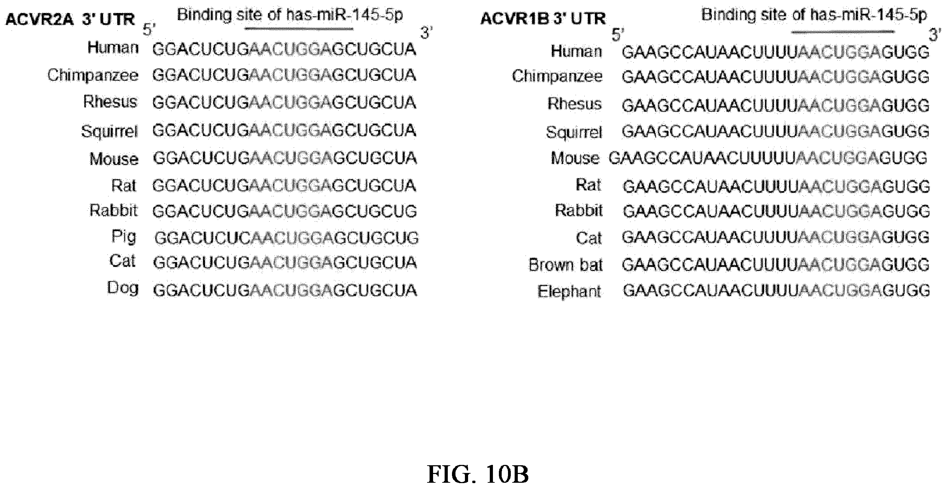

The result is shown in FIGS. 10A and 10B.

As can be seen from FIG. 10A, the seed sequence of has-miR-145-5p perfectly matched the 3'-untranslated region (UTR) of ACVR2A and ACVR1B, confirming that the activin A receptor is a potential target of has-miR-145-5p. In addition, as can be seen from FIG. 10B, the potential binding site of has-miR-145-5p was highly conserved among the mammalian ACVR2A and ACVR1B genes. These results show that the has-miR-145-5p of the exosomes derived from tonsil mesenchymal stem cells can affect activin A receptor-mediated response in muscle.

### <Example 5> Restoration of muscular differentiation impairment induced by activin A by treatment with exosomes derived from tonsil mesenchymal stem cells

To confirm the effect of the exosomes derived from tonsil mesenchymal stem cells on the maintenance of muscle mass, muscular differentiation in C2C12 myoblasts was investigated.

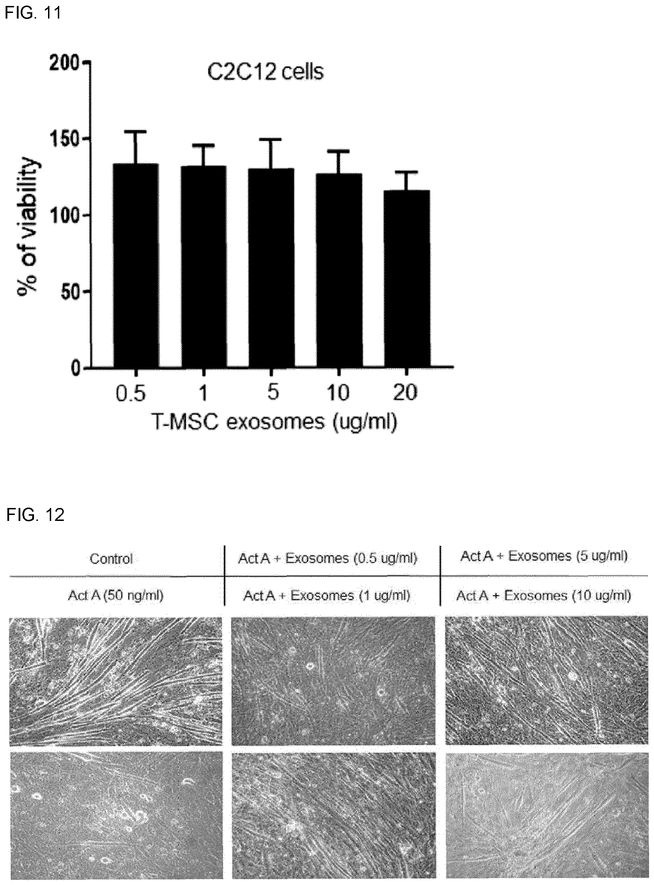

Prior to treating with the exosomes, potential toxicity was checked by measuring cellular viability and the result is shown in FIG. 11. As can be seen from FIG. 11, the C2C12 cells treated with the exosomes derived from tonsil mesenchymal stem cells at various concentrations had increased cell viability over 100%, as compared to untreated C2C12 cells set to 100%.

Next, it was tested whether the exosomes derived from tonsil mesenchymal stem cells improve muscular differentiation in the presence of activin A.

C2C12 myoblasts were treated with the 50 ng/mL activin A and the exosomes derived from tonsil mesenchymal stem cells at various concentrations (0.5, 1, 5 and 10 µg/mL) on the first day of differentiation. The result is shown in FIG. 12.

As can be seen from FIG. 12, the cells treated with activin A alone showed limited formation of myotubes after 5 days. Conversely, the cells exposed to the exosomes derived from tonsil mesenchymal stem cells formed abundant myotubes even in the presence of activin A.

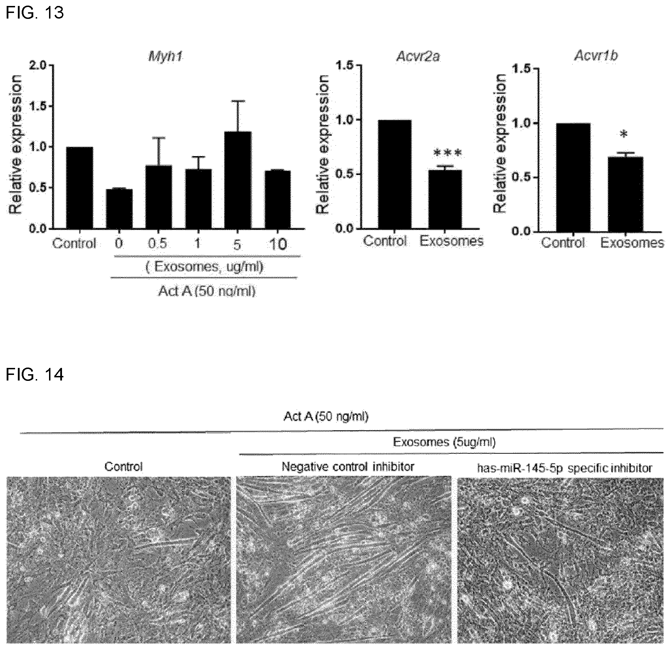

In addition, the change in the expression of the skeletal muscle markers MYH1, ACVR2A and ACVR1B was investigated by real-time PCR and the result is shown in FIG. 13.

The sequences of primers further used in addition to those described in the foregoing examples are as follows:

| Primer | Sequence | Size (bp) |
|---|---|---|
| *Acvr2a* | F: 5'-ataaacggcgacattgttttgc-3' (SEQ ID NO: 12) | 234 |
| | R: 5'-tcggtgtaacaggatttgaagtg-3' (SEQ ID NO: 13) | |
| *Acvr1b* | F: 5'-ctgcctacagaccaactacacc-3' (SEQ ID NO: 14) | 190 |
| | R: 5'-gcagaagtcaatatagcagcagt-3' (SEQ ID NO: 15) | |

As can be seen from FIG. 13, the expression of the skeletal muscle marker MYH1 was recovered in the C2C12 cells treated with the exosomes derived from tonsil mesenchymal stem cells under activin A exposure. In particular, the exosomes derived from tonsil mesenchymal stem cells most effectively increased the MYH1 expression at a concentration of 5 µg/mL.

In addition, considering the abundance of has-miR-145-5p in the exosomes derived from tonsil mesenchymal stem cells and the potential activity on activin A receptors, ACVR2A and ACVR1B, the expression of ACVR2A and ACVR1B in C2C12 cells treated with the exosomes derived from tonsil mesenchymal stem cells was investigated for 24 hours. The result showed that the expression of ACVR2A and ACVR1B was significantly downregulated in the C2C12 cells with the exosomes derived from tonsil mesenchymal stem cells added.

Thus, it was confirmed that the exosomes derived from tonsil mesenchymal stem cells can improve activin A-induced impaired muscular differentiation by enhancing muscular differentiation, particularly by regulating the expression of the activin A receptors.

### <Example 6> Regulation of muscular response to activin A by exosomes via has-miR-145-5p activity

In order to identify whether has-miR-145-5p plays a key role in the exosomes derived from tonsil mesenchymal stem cells supporting muscular differentiation, the change in myotube formation when a has-miR-145-5p-specific inhibitor (Bioneer, Daejeon) was used was investigated.

The result is shown in FIG. 14. As can be seen from FIG. 14, the treatment with the has-miR-145-5p inhibitor abrogated the supportive effect of the exosomes derived from tonsil mesenchymal stem cells on myotube formation.

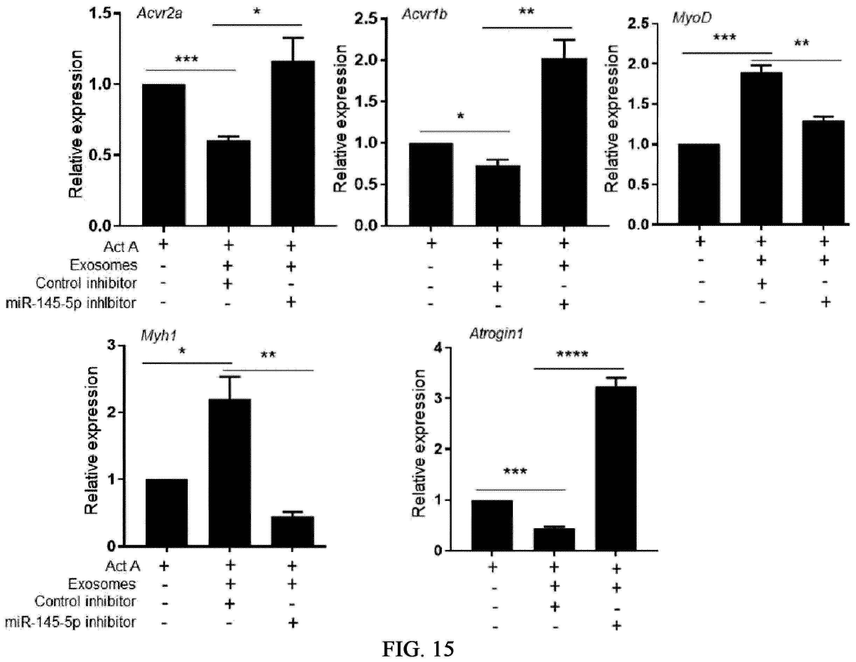

In addition, as can be seen from FIG. 15, the treatment with the has-miR-145-5p-specific inhibitor significantly reversed the expression of the ACVR2A, ACVR1B, MyoD and Myh1 genes induced by the exosomes derived from tonsil mesenchymal stem cells. Additionally, the Atrogin1 expression was downregulated by the exosomes derived from tonsil mesenchymal stem cells in a has-miR-145-5p-dependent manner.

These results correlate with the decreased expression of the activin A receptors, ACVR2A and ACVR1B, by the exosomes derived from tonsil mesenchymal stem cells via the has-miR-145-5p activity.

### <Example 7> Recovery of body weight loss and skeletal muscle loss in vivo by treatment with exosomes derived from tonsil mesenchymal stem cells

The exosomes derived from tonsil mesenchymal stem cells supplemented with a miR-145-5p-specific inhibitor were injected during Bu-Cy treatment and experiment was performed similarly to Example 2.

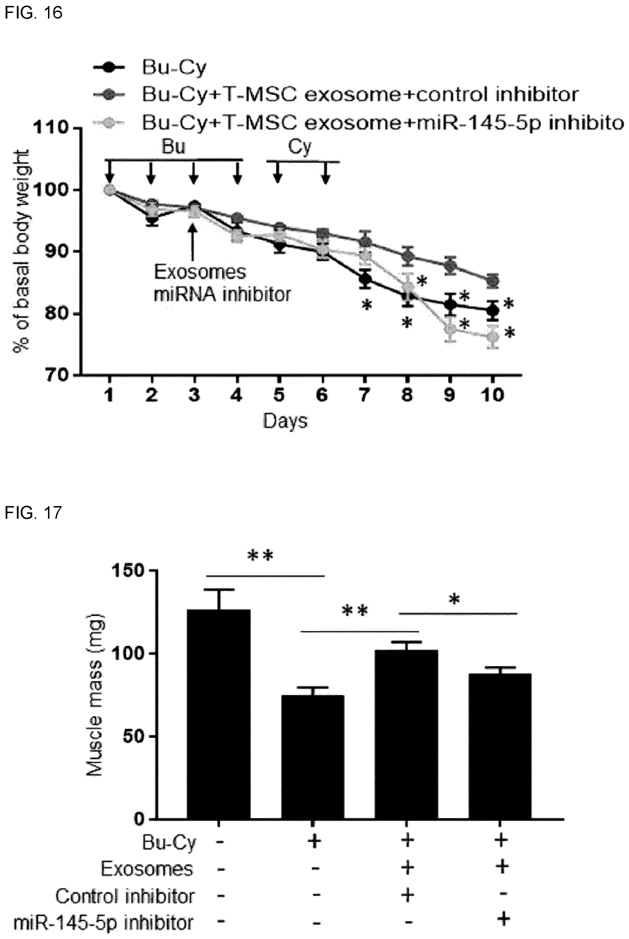

A result of investigating the change in body weight is shown in FIG. 16.

It was confirmed that the mice injected with the exosomes derived from tonsil mesenchymal stem cells and a nonspecific inhibitor (control) on the third day showed significantly inhibited body weight loss from the day after the last Cy treatment. However, these effects were diminished in the mice that administered the exosomes derived from tonsil mesenchymal stem cells and the miR-145-5p inhibitor starting from 2 days after the last Cy treatment. In particular, the co-administration of the exosomes derived from tonsil mesenchymal stem cells and the miR-145-5p inhibitor resulted in a sharp decrease in body weight towards the end of the experiment.

In addition, as can be seen from FIG. 17, the exosomes derived from tonsil mesenchymal stem cells used in combination with the has-miR-145-5p inhibitor also blunted the loss of muscle mass in each experimental mouse.

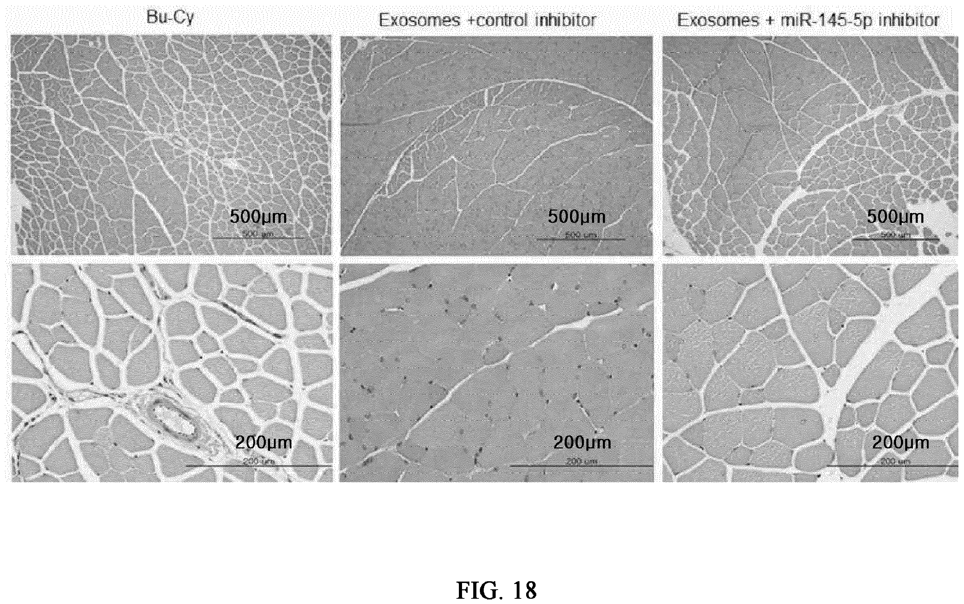

In addition, as can be seen from FIG. 18, the mice to which the exosomes derived from tonsil mesenchymal stem cells were injected showed minimized disruption of muscle fibers induced by Bu-Cy when the activity of miR-145-5p was maintained normally.

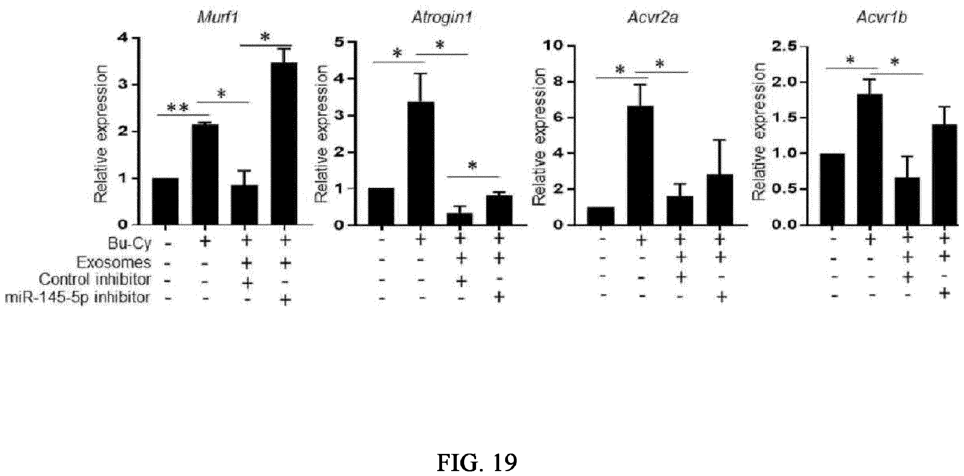

Lastly, as shown in FIG. 19, the co-administration of the has-miR-145-5p-specific inhibitor and the exosomes derived from tonsil mesenchymal stem cells to the Bu-Cy-treated mice reversed the expression of the MuRF1 and Atrogin1 atrophy genes. Also, the mice treated with Bu-Cy showed significant increase in the activin A receptors, ACVR2A and ACVR1B, indicating that activin A responsiveness can be augmented.

From the above results, it was confirmed that the exosomes derived from tonsil mesenchymal stem cells according to the present disclosure exhibit superior effect of improving musculoskeletal loss. In particular, it was confirmed that the expression level of has-miR-145-5p was higher as compared to the exosomes derived from other stem cells and it was highly correlated with the inhibited expression level of activin A. Based on these results, it was confirmed that the exosomes according to the present disclosure can be used as a therapeutic agent for a muscle loss-related disease.

## Claims

1. A pharmaceutical composition for preventing or treating a muscle loss-related disease, comprising exosomes derived from tonsil mesenchymal stem cells.

2. The pharmaceutical composition for preventing or treating a muscle loss-related disease according to claim 1, wherein the exosomes derived from tonsil mesenchymal stem cells comprise hsa-miR-145-5p of SEQ ID NO 1.

3. The pharmaceutical composition for preventing or treating a muscle loss-related disease according to claim 1, wherein the muscle loss-related disease indicates symptoms of muscle loss caused by decline in muscular function, muscle wasting or muscle degeneration.

4. The pharmaceutical composition for preventing or treating a muscle loss-related disease according to claim 1, wherein the muscle loss-related disease is one or more selected from a group consisting of sarcopenia, muscle atrophy, myasthenia, muscular dystrophy, myotonia, hypotonia, muscular weakness, amyotrophic lateral sclerosis and cachexia.

5. The pharmaceutical composition for preventing or treating a muscle loss-related disease according to claim 1, wherein the exosomes derived from tonsil mesenchymal stem cells have a diameter of 10-300 nm.

6. The pharmaceutical composition for preventing or treating a muscle loss-related disease according to claim 1, wherein the exosomes derived from tonsil mesenchymal stem cells inhibit the expression of activin A.

7. The pharmaceutical composition for preventing or treating a muscle loss-related disease according to claim 1, wherein the composition comprises 1×10⁷ to 1×10¹² exosomes/mL.

8. A food composition for preventing or alleviating a muscle loss-related disease, comprising exosomes derived from tonsil mesenchymal stem cells.

9. The food composition for preventing or alleviating a muscle loss-related disease according to claim 8, wherein the exosomes derived from tonsil mesenchymal stem cells comprise hsa-miR-145-5p of SEQ ID NO 1.

10. The food composition for preventing or alleviating a muscle loss-related disease according to claim 8, wherein the muscle loss-related disease is one or more selected from a group consisting of sarcopenia, muscle atrophy, myasthenia, muscular dystrophy, myotonia, hypotonia, muscular weakness, amyotrophic lateral sclerosis and cachexia.

11. A food composition for increasing muscle mass or improving muscular function, comprising exosomes derived from tonsil mesenchymal stem cells.

12. The food composition for increasing muscle mass or improving muscular function according to claim 11, wherein the exosomes derived from tonsil mesenchymal stem cells comprise hsa-miR-145-5p of SEQ ID NO 1.
